# EUROPEAN PATENT APPLICATION

(11) **EP 1 974 754 A1**
(43) Date of publication of application: **01.10.2008**
(21) Application number: 07706882.3
(22) Date of filing: 17.01.2007
(51) Int. Cl.: A61L 27/00

(54) **BIOMATERIAL FOR OSTEOGENESIS CONTAINING OSTEOGENESIS PROMOTER AND NANOGEL**

(30) Priority: 18.01.2006 JP 2006010072
(71) Applicant: National University Corporation Tokyo Medical and Dental University, Tokyo 113-8510 (JP)
(72) Inventor: AKIYOSHI, Kazunari, Tokyo 1600002 (JP); NODA, Masaki, Tokyo 1500022 (JP)
(74) Representative: De Clercq, Ann G. Y.
(86) International application number: PCT/JP2007/050555
(87) International publication number: WO 2007/083643

(57) **Abstract**

An object of the present invention is to provide an osteogenic biomaterial which achieves long-term sustained release and localized functional expression of a substance which promotes the formation of bone tissue.

The present inventors have found out that a nanogel efficiently encloses an osteogenesis promoting substance to suppress the substance from diffusion into blood, and that the nanogel functions as a carrier for local administration of the osteogenesis promoting substance. Furthermore, they have found out that the osteogenesis promoting substance-containing nanogel can be cross-linked with a water-soluble polymer to allow sustained release of the osteogenesis promoting substance at a local area over a long period of time. Based on these findings, the present invention was completed. The osteogenic biomaterial of the present invention which contains the osteogenesis promoting substance and the nanogel shows osteogenesis promoting activity over a long period of time, and can be used as a preventive and therapeutic agent against bone diseases.

## Description

### Technical field

The present invention relates to an osteogenic biomaterial containing an osteogenesis promoting substance and a nanogel.

### Background art

Bone is a tissue with a high repair and regenerative capacity, but a large damage caused by, for example, severe comminuted fractures and extraction of bone neoplasms, can not be completely repaired, and leaves co-ossification failure or bone loss. In addition, the aging population in recent years increases patients with osteoporosis, and give a proportionate increase in incidence of fractures from osteoporosis. These diseases cause functional impairment and pain, which bring obstacles to daily activities. Attempts have been made to regenerate bone tissue in order to cure these injuries, but have not yet succeeded in establishing a technology for forming bone specifically in a local area.

In recent years, substances such as cell growth factors, cytokines, and hormones involving in regeneration of bone tissue have been identified and attempted to apply to treatment. Known examples of these factors include a peptide osteogenesis promoting substance such as bone morphogenetic protein (BMP) or transforming growth factor (TGF). On the other hand, as the non-peptide osteogenesis promoting substance, prostaglandin E₂, EP4 agonist, prostaglandin A1 derivative, vitamin D3 derivative, benzylphosphonate derivative, and phenolsulfonphthalein derivative and the like have been reported.

Prostaglandin E₂ (henceforth abbreviated as PGE₂) which is a non-peptide osteogenesis promoting substance is a biologically active substance purified from arachidonic acid and functions in various tissues to keep homeostasis of the body. It has already been confirmed that PGE₂ can be administrated systemically and locally to various animals including humans to give osteogenesis, and is expected to use for clinical applications (non-patent document 1). However, it is known that PGE₂ has a short half-life in body, and thus must be administrated continuously for several times a day for a few weeks (non-patent document 2-4). In addition, it has a problem to cause a side-effect such as diarrhea. Therefore, there is desired to develop a technique wherein PGE₂ can be administrated to release in a controllable manner over a long period at a local area.

On the other hand, attention is paid particularly in the fields of drug delivery system and nanotechnology to a polymeric gel particle (nanogel) which has a nanometer size (in particular 100 nm or less) to combine both performances of a nano-sized fine particle and a gel. In general, chemically cross-linked nanogels have been synthesized by a microemulsion polymerization method or by an intramolecular cross-linking reaction. The present inventors have reported on a new preparation method for a physical cross-linking nanogel by the self-organization of a hydrophobic polymer (non-patent document 5). In other words, water-soluble polysaccharides, which are partially introduced with relatively high hydrophobic groups (cholesterol groups), have been found to associate in a self-organizing manner in their dilute aqueous solution, thereby to form a monodispersed nanogel with the association regions of the hydrophobic groups cross-linked.

A normal nano-sized fine particle is mostly researched to use exclusively its surface characteristics, while a nanogel additionally has an important characteristic of embracing a space for holding substances such as hydrophobic medicines or proteins. In particular, the nanogel of a cholesterol-bearing pullulan (CHP) functions as a host to interact selectively with proteins, and is reported to be effective as a carrier for a drug delivery system (non-patent document 6-8). Furthermore, cyclodextrin can be added to destroy the nanogel, facilitating release of the enclosed substances.

In addition, because it has a physical crosslinking point, it can be dynamically controlled in structure through crosslinking (Patent document 9). The hydrophobic group can be changed in structure to control the dynamic properties of the gel network, and the host-guest interaction with cyclodextrin can be used to control dynamically the creation and destruction of the nanogel. These properties have been successfully used to develop an artificial molecular chaperone to control the uptake and release of denatured proteins.
[Patent document 1] Publication No. WO WO00/12564
[Patent document 2] Japanese Patent Application Laid-open No. 3-232880
[Patent document 3] Japanese Patent Application Laid-open No. 4-364179
[Patent document 4] Japanese Patent Application Laid-open No. 5-294960
[Patent document 5] Japanese Patent Application Laid-open No. 8-231569
[Patent document 6] Japanese Patent Application Laid-open No. 7-29183 derivative
[Patent document 7] Japanese Patent Application Laid-open No. 8-73746
[Patent document 8] European Patent Publication No. 524023
[Non-patent document 1] Pilbeam, CC., Harrison, JR., Raisz, LG, In: Bilezikian,JP.,Raisz,LG.,Rodan, GA.,eds. Principles of bone biology. 2nd ed. San Diego: AcademicPress. vol. 2: p979-994.
[Non-patent document2] Suponitzky, I., Weinreb,M.J. Endocrinol. 156, 51-57 (1998).
[Non-patent document 3] Keila, S., Kelner, A.,Weinreb, M. J. Endocrinol.vol.168, p131-139 (2001).
[Non-patent document 4] Yoshida, K., Oida, H., Kobayashi, T.,Maruyama, T., Tanaka, M., Katayama, T., Yamaguchi, K., Segi,E., Tsuboyama, T.,Matsushita, M., Ito, K., Ito, Y., Sugimoto,Y.,Ushibuki, F., Ohuchida,S., Kondo,K., Nakamura, T., Narumiya,S.Proc.Natl. Acad. Sci. USA. vol. 99, p4580-4585(2002).
[Non-patent document 5] Akiyoshi, K., Deguchi,S.,Moriguchi, N., Yamaguchi, S., Sunamoto,J.Macromolecules. vol. 26, p3062-3068 (1993).
[Non-patent document 6] Akiyoshi, K., Taniguchi, I., Fukui, H., Sunamoto, J. Eur. J. Pharm. vol. 42, p286-290 (1996)
[Non-patent document 7] Akiyoshi, K., Kobayashi, S., Shichibe, S., Mix, D., Baudys,M.,Kim,S.W.,Sunamoto, J. J. Control. Release. vol. 54,p313-320(1998).
[Non-patent document 8] Ikuta, Y., Katayama, N., Wang, L., Okugawa,T.,Takahashi,Y.,Schmitt,M., Gu, X., Watanabe, M.,Akiyoshi,K.,Nakamura, H., Kuribayashi,K., Sunamoto,J.,Shiku,H. Blood. vol. 99, p3717-3724 (2002)
[Non-patent document 9] Morimoto, N., Endo, T., Iwasaki, Y., Akiyoshi,K.Biomacromolecules.vol. 6, p1829-1834 (2005)
[Non-patent document 10] Koshihara, Y, Takamori,R,Nomura, K, Sugiura, S, Kurozumi,S.Journal of Pharmacology Experimental Therapeutics, vol258, p1120-1126(1991)
[Non-patent document 11] Tenenbaum, HC, Torontali,M,Sukhu, B. Bone, vol13,p249-255 (1992)
[Non-patent document 12] Akedo, Y, Hosoi, T, Inoue S, Ikegami, A,Mizuno, Y, Kaneki, M, Nakamura,T, Ouchi, Y, Orimo, H. Biochemicaland Biophysical Research Communications, vol187,p814-820 (1992)

### Disclosure of the invention

### Problems to be solved by the invention

An object of the present invention is to provide an osteogenic biomaterial which allows a long-term release of an osteogenesis promoting substance to exhibit its functions in a local area.

### Means for solving the problems

Based on findings with regard to nanogels, the present inventors have found out that the nanogels can be used to develop a drug delivery system for osteogenesis promoting substances, and thus completed the present invention.

In other words, the present invention comprises:
1. An osteogenic biomaterial comprising at least an osteogenesis promoting substance and a polymeric gel fine particle (nanogel).
2. The osteogenic biomaterial according to claim 1, wherein the osteogenesis promoting substance is a non-peptide.
3. The osteogenic biomaterial according to claim 2, wherein the osteogenesis promoting substance is prostaglandin E₂.
4. The osteogenic biomaterial according to any of claims 1-3, wherein the nanogel is cholesterol-bearing pullulan (CHP).
5. The osteogenic biomaterial according to claim 4, wherein the CHP is introduced with an amino group or a carboxyl group.
6. The osteogenic biomaterial according to any of claims 1-5, wherein the nanogel is modified with a polymerizing group.
7. The osteogenic biomaterial according to claim 6, wherein the polymerizing group is an acryloyl group.
8. The osteogenic biomaterial according to any of claims 6 or 7, wherein the nanogel is cross-linked with a water-soluble polymer having a thiol group.
9. The osteogenic biomaterial according to claim 8, wherein the water-soluble polymer having a thiol group is polyethyleneoxide.
10. The osteogenic biomaterial according to any of claims 1-9, wherein the osteogenesis promoting substance has a weight ratio of approximately 6 x 10⁻⁶ - 2 x 10⁻² relative to the nanogel.
11. The osteogenic biomaterial according to any of claim 1-7, wherein the nanogel has a particle diameter of approximately 20-500 nm.
12. The osteogenic biomaterial according to any of claims 1-11, wherein the osteogenic biomaterial is a sustained release formulation.
13. The osteogenic biomaterial according to any of claims 1-11, wherein the osteogenic biomaterial is an injection.
14. A bone disease preventive and/or therapeutic agent comprising an osteogenic biomaterial according to any of claims 1-11.
15. A method for producing the osteogenic biomaterial according to any of claims 1-13.

### Effect of the Invention

According to the present invention, the nanogel, which efficiently encloses the osteogenesis promoting substance to suppress it from dispersion into blood, functions as a suitable carrier for local administration of the osteogenesis promoting substance. In addition, the osteogenesis promoting substance-containing nanogel can be cross-linked with a water-soluble polymer to allow sustained release of the osteogenesis promoting substance in a local area for a long period. Furthermore, according to the present invention, even a small amount of osteogenesis promoting substance can be administrated to promote bone formation at a target site. Therefore, the present invention, which uses the hydrophobic polysaccharide nanogel as a carrier, is effective for locally sustained release of the osteogenesis promoting substance which otherwise would disperses quickly into blood, allowing promotion of specific bone formation at the target site. As a result, the method of the present invention is expected to apply to a clinical field as a new administration method for osteogenesis promoting substance.

### Description of the Preferred Embodiment

Unless stated otherwise, the technical and scientific terms used in the present description have the same meaning as commonly understood by a skilled in the art. The present invention is described in further detail in reference to the embodiments of the present invention. The following detailed description is only exemplary and does not limit the present invention.

### (osteogenesis promoting substance)

As an osteogenesis promoting substance, currently, non-peptide and peptide substances are known, and either one can be used in the formulation of the present invention. Examples for the non-peptide osteogenesis promoting substance include prostaglandin E₂ (non-patent document 1-4), sulfur-containing heterocyclic compounds or the salts thereof (patent documents 2-5), benzopyran derivatives (patent document 6), phosphonic acid derivatives or the salts thereof (patent document 7), prostaglandin A1 derivatives (non-patent document 10), benzylsulfonic acid derivatives (patent document 8), phosphonic acid (non-patent document 11), vitamin K2 derivatives (non-patent document 12). In addition, examples for peptide osteogenesis promoting substance include bone morphogenetic protein (BMP), fibroblast growth factor (FGF), platelet-derived growth factor (PDGF), transforming growth factor (TGF-β), insulin-like growth factor-1 and 2 (IGF-1,-2), and parathyroid hormone (PTH). Among these osteogenesis promoting substances, non-peptide osteogenesis promoting substances are preferred, and prostaglandin E₂ is more preferred. In addition, two or more of these osteogenesis promoting substances can be combined to use at an appropriate ratio.

### (nanogel)

The method for producing a nanogel of a hydrophobized polymer used in the present invention is known. For example, a method is disclosed in Patent document 1 (high purity hydrophobic group-containing polysaccharide and a production method thereof). According to this, in the first stage reaction in the production method, a hydroxyl group-containing hydrocarbon or a sterol having a carbon number of 12-50 is reacted with a diisocynate compound represented by OCN-R1NCO (in the formula, R1 is a hydrocarbon group having a carbon number of 1-50) to produce an isocyanate group-containing hydrophobic compound resulting from the reaction of one molecule of the hydroxyl group-containing hydrocarbon or the sterol having a carbon number of 12-50.

In the second stage reaction in the production method, the isocyanate group-containing hydrophobic compound obtained in the first stage reaction is further reacted with a polysaccharide to produce a hydrophobic group-containing polysaccharide with a hydrocarbon group or a sterol group having a carbon number of 12-50 contained as the hydrophobic group. The reaction product of this second stage reaction can be purified with a ketene base solution to produce a high purity hydrophobic group-containing polysaccharide. The polysaccharide to use is one or more kinds selected from the group consisting of a polymer that has a hydrophobic group substitution of dextrin, mannose, amylase, and the like; polylysine, polyglutamic acid, polyarginic acid, polyarginine, polyisopropyl acrylamide (PNIPAM), and MPC.

The preferable nanogel includes cholesterol-bearing pullulan (henceforth abbreviated as CHP, there are 1-10, preferably 1- several cholesterols for 100 monosaccharides of pullulan of molecular weight 108,000), and CHP derivatives. The properties of the hydrophobic polymer can be changed by changing the amount of cholesterol substitution depending on the size of the protein or its hydrophobicity. In order to control the hydrophobicity, it is suitable to introduce an alkyl group having a carbon number of 10-30, preferably about 12-20. The nanogel used in the present invention preferably has a particle diameter of 10-40 nm, preferably 20-30 nm. The example of a suitable CHP derivative is a CHP derivative with an amino group or carboxyl group introduced. Nanogel is already commercially available, and in the present invention, these commercial products can be used.

The present invention can use a CHP derivative, more specifically a CHP derivative with an amino group or a carboxyl group introduced.

### 1) CHP derivative with amino group introduced

The CHP derivative with amino group introduced is represented by the following general formula (I).
[Formula 1]
(Wherein n is an integer of 50-500, R1 is -(CH₂)m-NH₂, m is an integer of 0-10).

In general formula (I), there are 1-50, preferably 5-30 amino groups per 100 monosaccharides of CHP. The amino group may be an alkyl amino group, that is, an amino group substituted with an alkyl group. Examples of the alkyl amino group include the amino group substituted with a linear or branched C₁₋₆ alkyl group such as methylamino, ethylamino, propylamino, butylamino, pentylamino, hexylamino, dimethylamino, diethylamino, methylethylamino, dipropylamino, dibutylamino, and dipentyl amino. In addition, it may be substituted with an aromatic alkyl phenyl group or an alkyl benzyl group.

The protective group of the amino group includes all groups which can normally be used as a protective group for an amino group, including acyl groups such as trichloroethoxycarbonyl, tribromoethoxycarbonyl, benzyloxycarbonyl, p-nitrobenzylcarbonyl, o-bromobenzyloxycarbonyl, (mono-, di-, tri-) chloroacetyl, trifluoroacetyl, phenylacetyl, formyl, acetyl, benzoyl, tert-amyloxycarbonyl, tert-butoxycarbonyl, p-methoxybenzyloxycarbonyl, 3,4-dimethoxybenzyloxycarbonyl, 4-(phenylazo) benzyloxycarbonyl, 2-fluorofuryloxycarbonyl, diphenylmethoxycarbonyl, 1,1-dimethylpropoxycarbonyl, isopropoxycarbonyl, phthaloyl, succinyl, alanyl, leucyl, 1-adamantyloxycarbonyl, and 8-quinolyloxycarbonyl; an aralkyl group such as benzyl, diphenylmethyl and trityl; an arylthio group such as 2-nitrophenylthio and 2,4-dinitrophenylthio; an alkyl or aryl sulfonyl group such as methanesulfonyl and p-toluenesulfonyl; a dialkylamino-alkylidene group such as N,N-dimethylaminoethylene; an aralkylidene group such as benzylidene, 2-hydroxybenzylidene, 2-hydroxy-5-chlorobenzylidene, and 2-hydroxy-1-naphthylmethylene; a nitrogen-containing heterocyclic alkylidene group such as 3-hydroxy-4-pyridylmethylene; a cycloalkylidene group such as cyclohexylidene, 2-ethoxycarbonylcyclohexylidene, 2-ethoxycarbonyl cyclopentylidene, 2-acetylcyclohexylidene, and 3,3-dimethyl-5-oxycyclohexylidene; a diaryl or diaralkyl phosphoryl group such as diphenylphosphoryl, dibenzylphosphoryl; a substituted silyl group such as 5-methyl-2-oxo-2H-1,3-dioxole-4-yl-methyl.

### 2) CHP derivative with carboxyl group introduced

The CHP derivative with carboxyl group introduced is represented by the following general formula (II).
[Formula 2]
(Wherein n is an integer of 50-500, R2 is a -(CH₂)m-COOH, m is an integer of 0-10.)

In general formula (II), there are 1-50, preferably 5-30 carboxyl groups per 100 glucose monosaccharides of CHP. The carboxyl protective group includes all groups which can normally be used as a protective group for a carboxyl group, including alkyl groups such as methyl, ethyl, n-propyl, isopropyl, 1,1-dimethylpropyl, n-butyl, and tert-butyl; aryl groups such as phenyl and naphthyl; aryl alkyl groups such as benzyl, diphenylmethyl, trityl, p-nitrobenzyl, p-methoxybenzyl, and (p-methoxyphenyl)methyl; acyl-alkyl groups such as acetylmethyl, benzoylmethyl, p-nitrobenzoylmethyl, p-bromobenzoylmethyl, and p-methanesulfonylbenzoylmethyl; oxygen-containing heterocyclic groups such as 2-tetrahydropyranyl and 2-tetrahydrofuranyl; halogeno-alkyl groups such as 2,2,2-trichloroethyl; alkyl silyl alkyl groups such as 2-(trimethylsilyl) ethyl; acyloxyalkyl groups such as acetoxymethyl, propionyloxymethyl, and pivaloyloxymethyl; cycloalkyl groups such as phthalimidemethyl; alkoxy-alkyl groups such as methoxymethyl, methoxyethoxymethyl, and 2-(trimethylsilyl) ethoxymethyl; arylalkoxyalkyl groups such as benzyloxymethyl; alkylthio-alkyl groups such as methylthiomethyl and 2-methylthioethyl; arylthio-alkyl groups such as phenylthiomethyl; alkenyl groups such as 1,1-dimethyl-2-propenyl, 3-methyl-3-butenyl, and allyl; and substituted silyl groups such as trimethylsilyl, triethylsilyl, triisopropylsilyl, diethylisopropylsilyl, tert-butyldimethylsilyl, tert-butyldiphenylsilyl, diphenylmethylsilyl, and tert-butylmethoxyphenylsilyl.

The compounds of general formula (I) and (II) can be converted to their salts. The examples include salts of the amino group or the carboxyl group. Examples of the salts of the amino groups include the salts with mineral acids such as hydrochloric acid, hydrobromic acid, and sulfuric acid; the salts with organic carboxylic acids such as tartaric acid, formic acid, citric acid, trichloroacetic acid, and trifluoroacetic acid; and the salts with sulfonic acids such as methane sulfonic acid, benzene sulfonic acid, p-toluene sulfonic acid, mesitylene sulfonic acid, and naphthalene sulfonic acid. Examples of the salts of the carboxyl groups include the salts with alkali metals such as sodium, and potassium; the salts with alkali earth metals such as calcium and magnesium; the ammonium salts; and the salts with nitrogen-containing organic bases such as trimethylamine, triethylamine, tributylamine, pyridine, N,N-dimethylaniline, N-methylpiperadine, N-methylmorpholine, diethylamine, dicyclohexylamine, procaine, dibenzylamine, N-benzyl-β-phenethylamine, 1-ephenamine, and N,N'-dibenzylethylene diamine.

The CHP derivative of the present invention can be synthesized, for example, by a preparation method as shown below.

### Preparation method 1: CHP derivative with amino-group introduced

(1) CHP is reacted with a dimethylaminopyridine (at a 0.1 mole ratio with respect to glucose monosaccharide of CHP) in a solvent. Pyridine may be used instead of the dimethylaminopyridine. In addition, examples of the solvent to use in this reaction include dimethylsulfoxide/pyridine, and dimethylformamide/pyridine. This reaction is normally carried out at 20-30°C for 15 minutes to 2 hours, preferably at 25°C for 1 hour.
(2) 4-nitrophenylchloroformate (equal mole ratio to the glucose monosaccharide of CHP)/dimethyl sulfoxide solution of 0.5 times volume the solution obtained in (1) was slowly instilled and stirred. N,N' -carbonyl imidazole may be used instead of the 4-nitrophenyl chloroformate. This reaction is normally carried out at 0-5°C for 3 hours to 6 hours, and preferably at 0°C for 4 hours. This reaction changes the hydroxyl group of the CHP to the nitrophenyl ester to provide an activated CHP.
(3) The solution obtained in (2) is treated in 20 times volume of a solvent to precipitate again. This reaction is normally carried out at 5-30°C for 30 minutes to 12 hours, preferably at 5°C for 12 hours. Examples of the solvent used in this reaction include ethanol, and ethanol/diethyl ether (v/v=1/1). The precipitate is then collected, and dried in vacuum at an ordinary temperature.
(4) The vacuum dried precipitate obtained in (3) is dissolved in a dimethyl sulfoxide/pyridine mixture solvent, and 0.03 times volume of ethylene diamine/dimethyl sulfoxide/pyridine solution is slowly instilled and stirred. This reaction may be normally carried out at 20-30°C for 3-5 days, preferably 25°C for 4 days. This reaction changes the hydroxyl group of the CHP to the carbamate ester.
(5) The solution obtained in (4) is treated in 20 times volume of a solvent to precipitate again. This reaction is normally carried out at 5-30°C for 30 minutes to 12 hours, preferably at 5°C for 12 hours. Examples of the solvent used in the reaction include ethanol, and ethanol/diethyl ether (v/v=1/1). The precipitate is then collected, and dried in vacuum at an ordinary temperature.
(6) The vacuum dried precipitate obtained in (5) is dissolved in dimethyl sulfoxide, dialyzed against distilled water, dialyzed against a sodium hydroxide solution (pH 12.8), then neutralized with hydrochloric acid, further dialyzed against distilled water, and freeze dried. Normally, dialysis may be conducted at 20-25°C for 5-8 days, preferably for 7 days at 20°C.

### Preparation method 2: CHP derivative with carboxyl introduced

(1) CHP and dimethylaminopyridine (at a 0.1 mole ratio relative to glucose monosaccharide of CHP) are reacted in a solvent. Examples of the solvent used in this reaction include dimethyl sulfoxide/pyridine, and dimethylformamide/ pyridine. The reaction is normally carried out at 20-30°C for 15 minutes to 2 hours, preferably at 25°C for 1 hour.
(2) 4-nitrophenyl chloroformate (equal mole ratio to the glucose monosaccharide of CHP)/dimethyl sulfoxide solution of 0.5 times volume the solution obtained in (1) is slowly instilled and stirred. N,N'-carbonyl imidazole may be used instead of the 4-nitrophenyl chloroformate. This reaction is normally carried out at 0-5°C for 3 hours to 6 hours, and preferably at 0°C for 4 hours. This reaction changes the hydroxyl group of the CHP into the nitrophenyl ester to obtain an activated CHP.
(3) β-alanine ethyl ester hydrochloride of 2.5 times weight the CHP is added to the solution obtained in (2). This reaction is normally carried out at 20-30°C for 3 days to 5 days and preferably at 25°C for 4 days. This reaction changes the hydroxyl group of CHP into the carbamate ester.
(4) The solution obtained in (3) is treated in 20 times volume of a solvent to precipitate again. This reaction is normally carried out at 5-30°C for 30 minutes to 12 hours and preferably at 5°C for 12 hours. Examples of the solvent used in this reaction include ethanol, ethanol/diethyl ether (v/v=1/1), and ethanol/diethyl ether (v/v=8/2). The precipitate is then collected, and dried in vacuum at an ordinary temperature.
(5) The vacuum dried precipitate obtained in (4) is dissolved in dimethyl sulfoxide, dialyzed against distilled water, dialyzed against a sodium hydroxide solution (pH 12.8) to de-protect the ethyl group, further dialyzed against distilled water, and then freeze-dried. Normally, dialysis may be carried out at 20-25°C for 5-8 days and preferably at 20°C for 7 days.

### (Preparation of a formulation containing osteogenesis promoting substance and nanogel)

One concrete example of a method for producing the formulation of the present invention is described below.
(1) Nanogel is dispersed in physiological saline to give approximately 1-30 mg/ml, preferably approximately 10-20 mg/ml.
(2) The osteogenesis promoting substance solution is prepared. The osteogenesis promoting substance is dissolved in physiological saline (0.153 mol/l of NaCl). This physiological saline generally contains approximately 0.0006-2.4 vol/% and preferably approximately 0.006-0.02 vol/% of ethanol and generally contains approximately 0.0002-0.8 vol/% and preferably approximately 0.002-0.007 vol/% of a surface active agent. The preferable surface active agent is Tween-80 and the like.
(3) The osteogenesis promoting substance solution obtained in (2) is diluted with the nanogel solution obtained in (1), and then left to stand generally at 4-37°C and preferably at 4°C, generally for 12-24 hours and preferably for 24 hours. The nanogel has different mix ratios to osteogenesis promoting substance depending on the type and target of the nanogel and the osteogenesis promoting substance, and generally has a weight ratio of approximately 6 x 10⁻⁶ to 2 x 10⁻², preferably approximately 6 x 10⁻⁶ to 2 x 10⁻⁴ relative to the osteogenesis promoting substance. The osteogenesis promoting substance has different contents in the mixture solution depending on the type and target of the osteogenesis promoting substance, and generally is adjusted to have a final concentration of approximately 0.06 to 60 µg/ml, preferably approximately 0.1 to 0.6 µg/ml.

### (Preparation of hydrogel with osteogenesis promoting substance-containing nanogel cross-linked)

The osteogenesis promoting substance-containing nanogel, in which an osteogenesis promoting substance is contained in a polymerizing nanogel, can be cross-linked with a water-soluble polymer having a thiol group at the terminus through a Michael addition reaction under a physiological condition, thereby to form a hydrogel. The hydrogel with osteogenesis promoting substance-containing nanogel cross-linked has high effects in sustained release and stability, allowing sustained release of the osteogenesis promoting substance over a long time at a local site.

The polymerizing nanogel is obtained by modifying a nanogel with a polymerizing group (suitably such as acroyl group, methacroyl group, and vinyl sulfone group). The nanogel has different modification rates with the polymerizing group depending on the type of nanogel and polymerizing group, and generally has a rate of 10-30/100 monosaccharides, and preferably 20-30/100 monosaccharides. One concrete example of a method for preparing a hydrogel with an osteogenesis promoting substance-containing nanogel cross-linked is described below.
(1) The nanogel is modified with a polymerizing group. The nanogel (generally 0.25-2 g, preferably 0.5-1g) and N,N'-dimethylaminopyridine (generally 5-40 ml, preferably 10-20 ml) are dissolved in an organic solvent. The organic solvent differs in type depending on the type of the nanogel and the polymerizing group, and includes dimethylsulfoxide (DMSO), and dimethylformamide. Preferably, DMSO is used. The nanogel in the organic solvent solution differs in concentration depending on the type of the nanogel and the organic solvent, and in general has a concentration of approximately 10-100 mg/ml, and preferably approximately 50-100 mg/ml.
(2) An Osteogenesis promoting substance solution is prepared. The osteogenesis promoting substance is dissolved in a physiological saline (0.153 mol/l of NaCl) . The physiological saline generally contains approximately 0.0006-2.4 vol/%, and preferably approximately 0.006-0.02 vol/% of ethanol and generally contains approximately 0.0002-0.8 vol/%, and preferably approximately 0.002-0.007 vol/% of a surface active agent. The preferable example of this surface active agent is Tween-80 and the like.
(3) The osteogenesis promoting substance solution obtained in (2) is diluted with the polymerizing nanogel solution obtained in (1), and then left to stand generally at 4-37°C and preferably at 4°C, generally for 12-24 hours and preferably for 24 hours. The polymerizing nanogel has different mix ratios to the osteogenesis promoting substance depending on the type and target of the nanogel and the osteogenesis promoting substance, and generally has a weight rate of approximately 0.0001 to 0.02, preferably approximately 0.001 to 0.02 relative to the osteogenesis promoting substance. The osteogenesis promoting substance has different contents in the mixture solution depending on the type and target of the osteogenesis promoting substance, and generally has a final concentration of approximately 2 to 400 µg/ml, preferably approximately 20 to 400 µg/ml.
(4) The water-soluble polymer having a thiol group is dissolved in a physiological saline to prepare a water-soluble polymer solution. The thiol group includes an alkyl thio group, an aralkyl thio group, and an acyl thio group. Preferable examples of the water-soluble polymer having a thiol group at its terminal include polyethylene oxide, hyaluronic acid, MPC, and polyacrylic acid.
(5) The osteogenesis promoting substance-containing polymerizing nanogel obtained in (3) is mixed with the water-soluble polymer solution obtained in (4) in such a rate that the polymerizing group of the former may be equal in mole number to the water-soluble polymer of the latter. Approximately 2-10 µl, and preferably approximately 2-5 µl of the resulting mixture solution is instilled on the Parafilm (registered trademark), and then left to stand generally at 4-37°C, and preferably at 20-37°C, generally for approximately 15 minutes to 3 hours, and preferably for approximately 30 minutes to 2 hours, thereby to give a hydrogel with osteogenesis promoting substance-containing polymerizing nanogel cross-linked. This mixture can be formulated to contain the osteogenesis promoting substance-containing polymerizing nanogel at a single dose of generally approximately 0.06-12 µg and preferably approximately 0.6-12 µg.

The formulation of the present invention can be prepared by a suitable method into a parenteral formulation for local administration (for example, an intramuscular or subcutaneous injection, an injection into organ or joint, an implant, a solid formulation such as granule or powder, and a liquid formulation such as suspension).

As one concrete example for an injection, the osteogenesis promoting substance-containing nanogel can be treated together with a dispersant (including a surface active agent such as Tween 80 and HCO-60; a polysaccharides such as carboxymethylcellulose, sodium alginate, and hyaluronic acid; and polysorbate), a preservative (such as methylparaben and propylparaben), an isotonization agent (such as sodium chloride, mannitol, sorbitol, and glucose), a buffer (such as calcium carbonate), and a pH adjusting agent (such as sodium phosphate, and potassium phosphate) to give an aqueous suspension, thereby to prepare a practical injection formulation. Alternatively, the above nanogel can be mixed with a vegetable oil such as sesame oil and corn oil or a phospholipid such as lecithin, or dispersed with a middle chain fatty acid triglyceride to prepare an oil-based suspension, thereby to prepare a practicable injection.

The formulation of the present invention may contain phosphoric acid or a salt thereof (such as sodium phosphate and potassium phosphate), and can be supplied with a phosphate to enhance the osteogenesis promoting action. The injection contains phosphoric acid or a salt thereof to get its concentration of generally approximately 0.1 mM-500 mM, and preferably approximately 1 mM-100 mM in the injection. Furthermore, low molecular weight polyethylene glycol and the like may be mixed as desired.

The formulation of the present invention, which has high sustained release and stability for the osteogenesis promoting substance, can be administrated once to promote bone formation. It can be used to prevent and treat bone diseases (including fracture, refracture, osteoporosis, Behcet's disease, ankylosing spondylitis, chronic rheumatoid arthritis, osteoarthritis, and the destruction of a joint tissue in their similar diseases), to repair bone tissue after surgery against multiple myeloma, lung cancer, breast cancer and the like, and to regenerate a periodontal tissue in periodontal disease and the like.

In addition, the formulation of the present invention has low toxicity and can be used safely in mammals (for example, human, cattle, horse, pig, dog, cat, mouse, rat, and rabbit). The formulation of the present invention may contain different doses depending on the type and content of the osteogenesis promoting substance, dosage form, duration of drug release, and target animal, as long as they are effective doses of the osteogenesis promoting substance.

### [Example]

The present invention will be described with reference to examples, but the examples are only exemplary for the present invention, and do not limit the technical scope of the present invention.

### Example 1

### <Synthesis of acryloyl group modified CHP (CHPA)>

The nanogel (CHP) was modified with a polymerizing group (acryloyl group) to synthesize the polymerizing nanogel (acryloyl group-modified CHP).

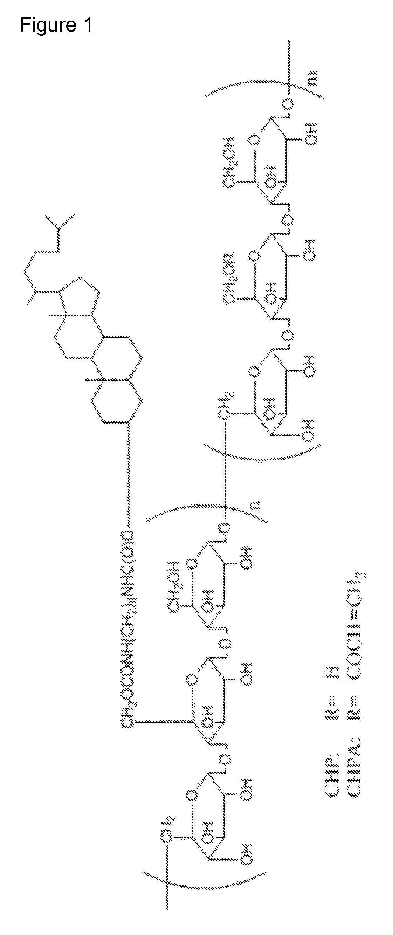

Cholesterol bearing pullulan (CHP) (0.5 g, monosaccharide number 2.98 mmol) (molecular weight of pullulan 108 x 10³, containing 1.4 cholesteryl group for 100 monosaccharides) and N,N'-dimethylaminopyridine (0.01 g, 0.0833 mmol) (made by Wako Co.) were dissolved in 5 ml of 99.0% dimethylsulfoxide (DMSO) (made by Wako Co.). Acrylic acid (204-613 µl, 298-894 mmol) (made by Nacalai tesque Co.) and the equal mole of N,N'-dicyclohexylcarbodiimide (made by Kanto Kagaku Co.) were dissolved in 5 ml of 99.0% DMSO, and then stirred for 30 minutes at room temperature. The acrylic acid solution was added to the CHP solution, and then stirred for 2 days at room temperature. The reactant was instilled into an excess amount of a mixture solvent of ether and ethanol (mixture ratio 85:15 to 100:0) to precipitate a polymer. The resulting precipitate was dissolved in 99.0% DMSO, then dialyzed against distilled water for 7 days, and freeze dried to give CHPA. The acryloyl group modification rate was determined by 1H-NMR. The resulting CHPA had an acryloyl group modification rate of 6-30 per 100 monosaccharides. The chemical structural formula for CHP and CHPA are shown in Fig. 1.

### Example 2

### <Preparation of PGE₂-containing hydrophobized polysaccharide nanogel>

Prostaglandin E₂ (PGE₂) was embedded in a nanogel (CHP) to prepare a PGE₂-containing hydrophobized polysaccharide nanogel.

As the hydrophobized polysaccharide, cholesterol bearing pullulan (CHP) (pullulan molecular weight of 108X10³, 1.4 cholesteryl groups for 100 monosaccharides) was used. The CHP nanogel was dispersed in physiological saline (0.153 mol/l NaCl) to have 20-30 mg/ml. PGE₂ was dissolved in physiological saline containing 3 vol% of ethanol and 1 vol% Tween 80 to have 3 mg/ml. The resulting aqueous PGE₂ solution was diluted with a CHP nanogel solution to have a PGE₂ final concentration of 0.06-60 µg/ml, and then left to stand overnight at 4°C to obtain a PGE₂-containing CHP nanogel.

### Example 3

### <Synthesis of hydrogel with PGE₂-containing hydrophobized polysaccharide nanogel cross-linked>

A polymerizing group such as acryloyl group or methacryloyl group will bond to a thiol group through a Michael addition reaction under physiological conditions. Thus, a CHP nanogel was crosslinked with a water-soluble polymer to synthesize a hydrogel. More specifically, PGE₂ was embedded in the CHP which had been modified by a polymerizing group (acryloyl group) (a polymerizing CHP) to give a PGE₂-containing CHP nanogel, which was then cross-linked with a water-soluble polymer having a terminal thiol group to synthesize a hydrogel with PGE₂-containing hydrophobized polysaccharide nanogel cross-linked.

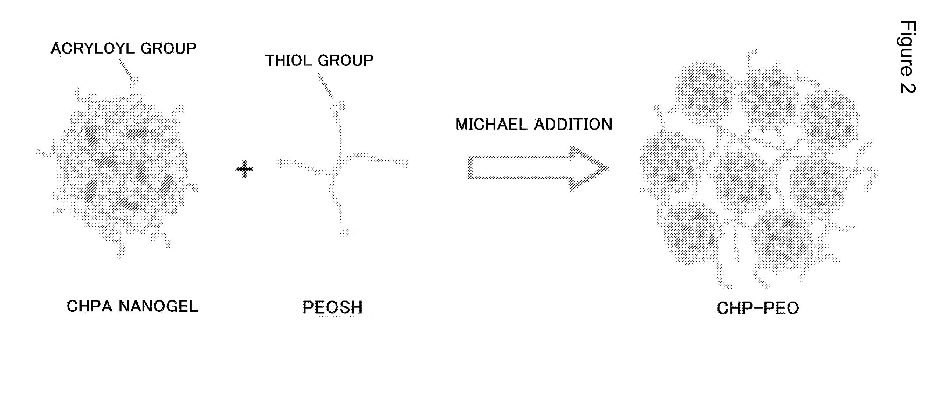

The polymerizing CHP nanogel prepared by the same method as in Example 1 was dispersed in physiological saline to have a concentration of 15 mg/ml or greater. PGE₂ was dissolved to have 75 mg/ml in physiological saline containing 75 vol% ethanol and 25 vol% Tween 80. This PGE₂ solution was diluted with the polymerizing CHP nanogel solution to have a final PGE₂ concentration of 0.15 to 3 mg/ml, and left to stand overnight at 4°C to obtain the PGE₂-containing polymerizing CHP nanogel. As the water-soluble polymer having a terminal thiol group, polyethylene oxide (PEOSH) (manufactured by Nihon Yushi Co.) was selected. This was dissolved in physiological saline, then supplied with the PGE₂-containing polymerizing CHP nanogel solution, and stirred. Here, they were mixed in such a rate that the polymerizing group of the polymerizing CHP might be equal in mole number to the thiol group of the water-soluble polymer. 5 µl of the resulting mixture solution was instilled on the Parafilm (registered trademark) (manufactured by Pechiney Plastic Packaging Com.), and then left to stand for 10 minutes to 3 hours at 37°C to obtain a hemispherical hydrogel with PGE₂-containing CHP cross-linked. A schematic drawing of the synthesis of a hydrogel with CHP nanogel cross-linked is shown in Fig. 2.

### Example 4

### <Osteogenesis in a mouse parietal bone using PGE₂-containing hydrophobized polysaccharide nanogel>

PGE₂-containing hydrophobized polysaccharide nanogel was administered to a mouse parietal bone to observe osteogenesis.

The PGE₂-containing CHP prepared by the same manner as in Example 2 was locally administrated onto the parietal bone of a 3-week old ICR mouse 5 times a week for 4 weeks. 10 ml of the prepared PGE₂-containing CHP solution (PGE₂ 0.6 µg/ml) was administered for a dose. Evaluation was conducted for 4 groups of physiological saline, PGE₂, CHP, PGE₂-contaning CHP (N=5). After 4 weeks of administration, the mice were sacrificed, and the parietal bones were collected, and the width of the parietal bone was measured by 2-D µCT. In order to study the effect on the bones of a whole body, the amount of cancellous bone of the femur was evaluated.

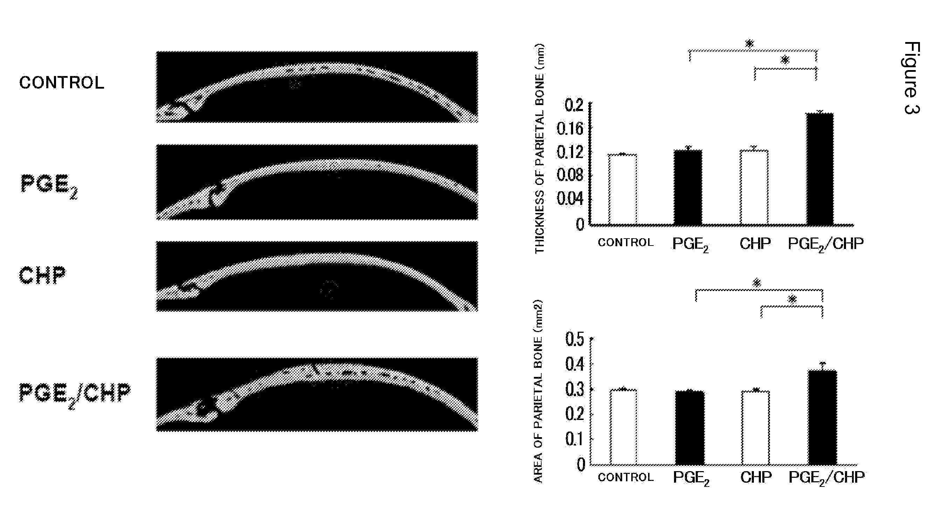

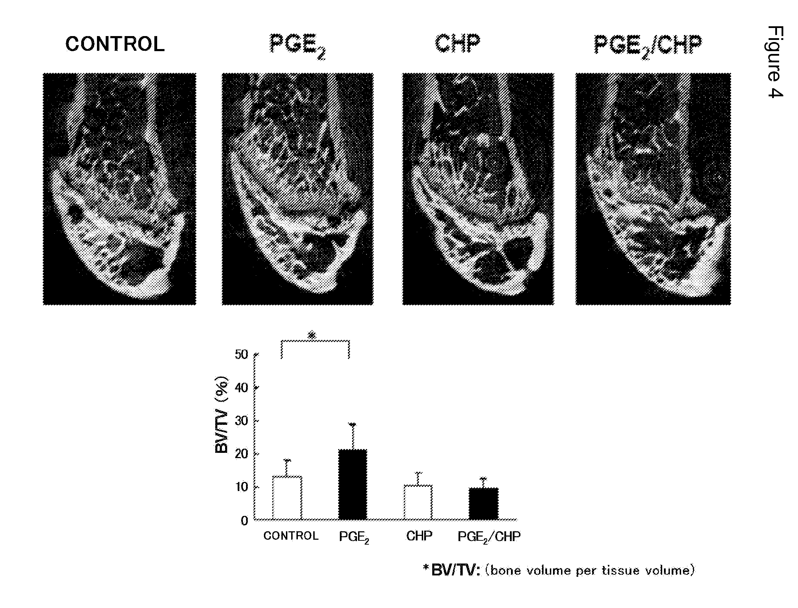

As a result, as shown in Fig. 3, the PGE₂-containing CHP nanogel was administrated to increase the width of the parietal bone. In contrast, physiological saline, PGE₂ only or CHP nanogel only was administrated to show no change. In addition, as shown in Fig. 4, PGE₂ only was administrated to increase the amount of cancellous bone in the femur apart from the administration site, while PGE₂-containing CHP nanogel was administrated to show no increase in amount of the same bone as above-mentioned.

From these results, it was revealed that the CHP nanogel enclosed PGE₂ to allow efficient sustained release of the enclosed PGE₂. In addition, it was demonstrated that PGE₂ only was administrated to diffuse readily in blood, causing side-effects, while the PGE₂ enclosed in the CHP was administrated to allow the PGE₂ to be suppressed from excessive diffusion, thereby to exhibit the localized action at the administration site.

### Example 5

### <Evaluation of hydrophobized polysaccharide nanogel on the ability to enclose and sustained-release PGE₂>

In order to understand the mechanism at the cellular level of the PGE₂-containing CHP nanogel, the osteoblast cell line MC3T3E1 cell culture system was used to analyze the cell differentiation by alkaline phosphatase (ALP) activity and the cell growth by the MTT method.

MC3T3E1 cells were seeded 5x10³ cells/well in a 96-well plate and cultured in the presence of 0.5% CO₂ at 37°C for 1 day. These cells were supplied with the PGE₂-containing CHP solution (PGE₂ 10 µM) prepared in the same manner as in Example 2 to get the same concentration, then cultured in the presence of 0.5% CO₂ at 37°C for 24 hours, then collected by scraping, and dissolved in 0.05 ml of a lysis buffer. The alkaline phosphatase (ALP) activity was measured according to the published protocols. In addition, the MTT method was conducted according to the published protocols.

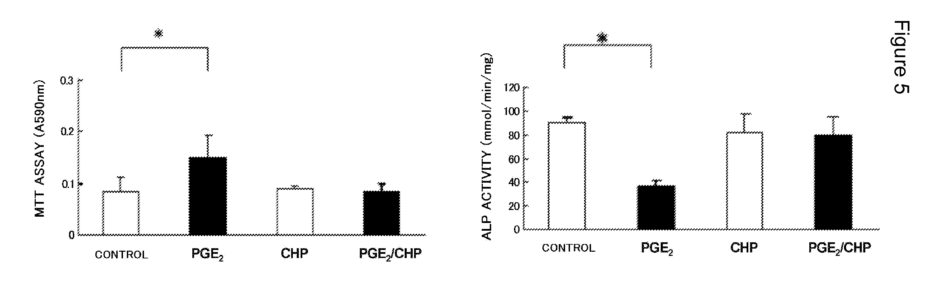

As a result of measurement of ALP activity and evaluation by the MTT method, as shown in Fig. 5, PGE₂ only was added to the cells to increase cellular differentiation and growth. In contrast, the PGE₂-containing CHP nanogel was added to exhibit no change in both cellular differentiation and growth. This is thought to be because PGE₂ was efficiently trapped by the CHP nanogel to sustained-release, allowing PGE₂ to keep a low concentration in the culture solution. From these results, it is suggested that the CHP nanogel allows sustained release of PGE₂ for a long term.

### Example 6

### <Osteogenesis in mouse parietal bone using hydrogel with PGE₂-containing hydrophobized polysaccharide nanogel cross-linked>

In order to study whether or not the PGE₂-containing hydrophobized polysaccharide nanogel can be cross-linked to promote the sustained-release, thereby to decrease the administration number and the dosage amount, the hydrogel with PGE₂-containing hydrophobized polysaccharide nanogel cross-linked was administered to the mouse parietal bone to evaluate osteogenesis.

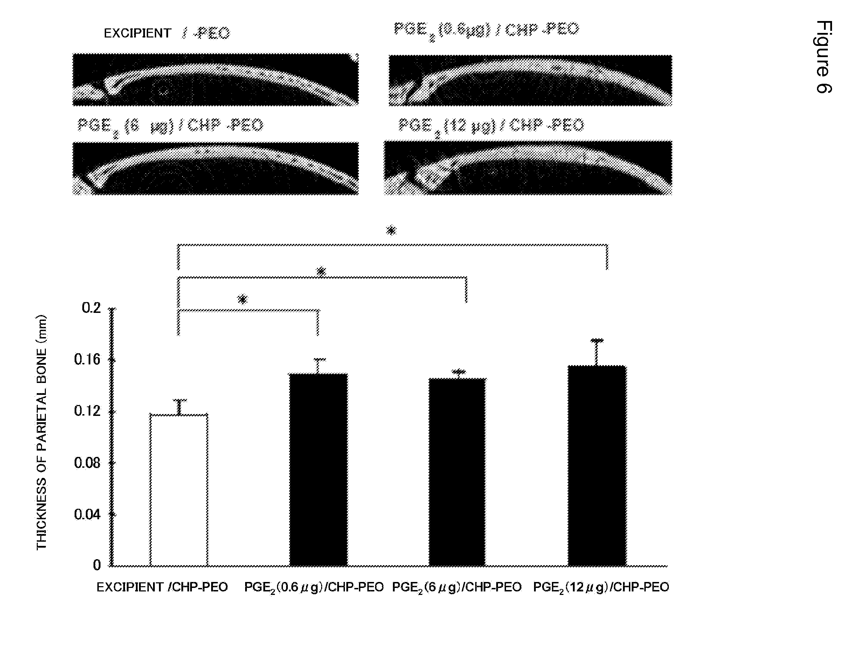

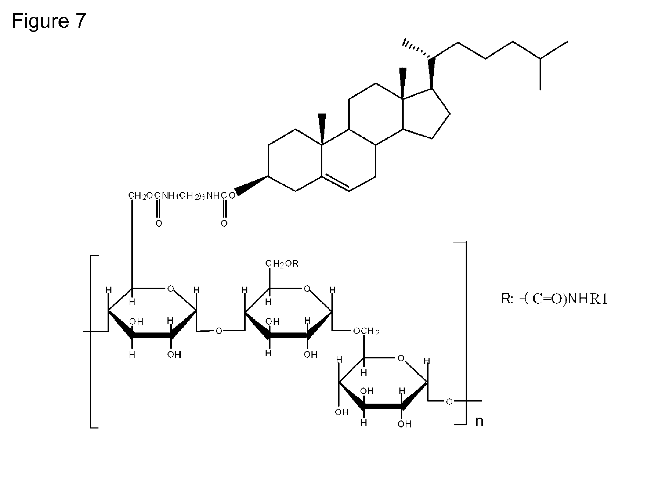

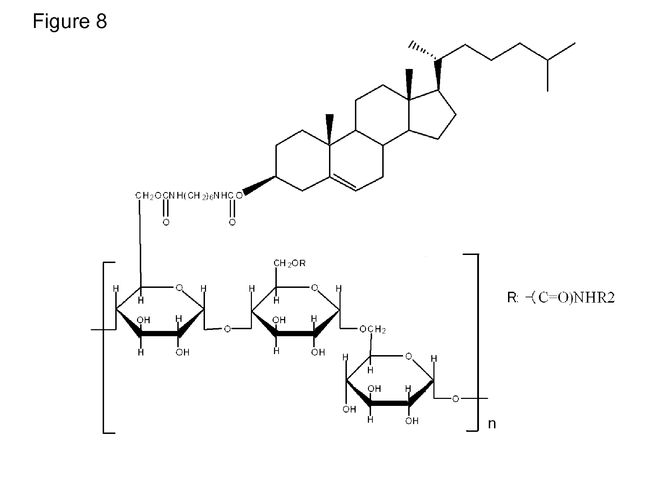

The PGE₂-containing CHP hydrogels, which were prepared by the same method as in Example 3 to contain 0. 6, 6, and 12 µg of PGE₂, were embedded once on the parietal bones of 3 week-old ICR mice. They were sacrificed 4 weeks after administration to collect the parietal bones, and the widths of the parietal bones were measured by 2 dimensional µCT. As a result, as shown in Fig. 6, an increase in width of parietal bone was seen. This reveals that the CHP nanogel can be cross-linked to improve sustained-release of PGE₂ from the CHP nanogel, thereby to control over a long period.

### Example 7

Bone morphogenetic protein (BMP) has been identified as a molecule which induces heterotopic osteogenesis and has been recognized to be effective for bone healing. However, it has been reported that higher animals require BMP-2 100 times or greater as much as mouse or rat to induce new bone formation. There have been many reports on delivery systems of BMPs to solve the above problem, but many of them use disadvantageously antigenic scaffolds or cultured cells which are used in vivo to cause a risk such as an immune reaction.

Cholesterol-bearing pullulan (CHP) nanogel has a chaperone activity and has been reported to be useful for a protein delivery system. Thus, we used CHP as a carrier for rhBMP-2 to investigate new bone formation and bone healing.

### Synthesis of hydrogel with BMP-2 (2mg)-containing hydrophobized polysaccharide nanogel cross-linked

The CHPA nanogel was dispersed in PBS to get 30-50 mg/ml. 2 ml of 1 mg/mol BMP-2 solution was added to 2 ml of the CHPA solution, and left to stand overnight at 4°C to make the BMP-2 enclosed in the CHPA nanogel. PEOSH [Pentaerythritol tetra (mercaptoethyl) polyethylene] was dissolved in PBS, and 1 ml of the PEOSH solution was added to 4 ml of the BMP-2-enclosing CHPA nanogel solution, and then quickly stirred. Here, the acryloyl group of the CHPA was adjusted to be equal in mole to the thiol group of the PEOSH. 5 ml of the resulting mixture solution was instilled on the Parafilm (product name), and then left to stand at 37°C for 2 hours to obtain a hemispherical hydrogel with BMP-2-containing CHPA cross-linked. In addition, the BMP-2 was diluted in PBS to get a BMP-2 content of 2 mg or less and used.

When CHP alone or BMP (2 µg)/CHP was implanted onto a mouse temporal bone periosteum, new bone formation was induced in the BMP/CHP group. Formation of the new bone began up to the fifth day after surgery, and it was seen that cells were induced to form a bone, and that a structure was formed for myeloid tissue to be accompanied by blood vessel formation in the new bone. Next, in order to investigate the ability of BMP/CHP to induce formation of new bone in a bone lacking site, a 4.6 mm diametric bone lacking site was prepared in a mouse skull to implant with CHP only or BMP (2 µg)/CHP. After 4 weeks, the CHP group showed almost no bone repair in the bone lacking site, while the BMP/CHP group showed almost complete bone repair.

Finally, in order to investigate the release of BMP-2 from CHP, MC3T3-E1 cells, which were osteoblast-like strains, were treated with rhBMP-2 and BMP/CHP to compare their ALP activities. The rhBMP-2 group showed an increase in ALP activity in a BMP dose-dependent manner, while the BMP/CHP group showed suppression of the BMP2-induced ALP activity from increasing. It is thought that the BMP/CHP group showed reduction in ALP activity because little BMP-2 is released from CHP, suggesting that CHP has strong ability to retain BMP-2. From the above, the possibility is revealed that CHP is useful as a scaffold for a delivery system for BMP and effective for bone formation at a bone lacking site.

### Industrial applicability

As described above, the osteogenic biomaterial of the present invention, which uses a nanogel material and contains an osteogenesis promoting substance, advantageously allows effective sustained-release of a drug at a local area and promotes specific bone formation at the target site. It is expected to apply clinically as a novel method for administrating the osteogenesis promoting substance.

### Brief description of the drawings

Fig. 1 shows a chemical structural formula of CHP and CHPA. (Example 1)
Fig. 2 shows a schematic diagram of the synthesis of a CHP nanogel crosslinked hydrogel. (Example 3)
Fig. 3 shows osteogenesis in the parietal bone resulting from the administration of PGE₂-containing CHP nanogel. (Example 4)
Fig. 4 shows the increase in cancellous bone amount in the femur resulting from the administration of PGE₂-containing CHP nanogel. (Example 5)
Fig. 5 shows the results of evaluating the cell growth and differentiation resulting from the addition of PGE₂-containing CHP nanogel. (Example 5)
Fig. 6 shows the osteogenesis in the parietal bone resulting from the administration of hydrogel with PGE₂-containing CHP nanogel crosslinked. (Example 6)

## Claims

1. An osteogenic biomaterial comprising at least an osteogenesis promoting substance and a polymeric gel fine particle (nanogel).

2. The osteogenic biomaterial according to claim 1, wherein the osteogenesis promoting substance is a non-peptide.

3. The osteogenic biomaterial according to claim 2, wherein the osteogenesis promoting substance is a prostaglandin receptor modulating agent.

4. The osteogenic biomaterial according to any of claims 1-3, wherein the nanogel is cholesterol-bearing pullulan (CHP) and the derivative thereof.

5. The osteogenic biomaterial according to claim 4, wherein the CHP is introduced with an amino group or a carboxyl group.

6. The osteogenic biomaterial according to any of claims 1-5, wherein the nanogel is modified with a polymerizing group.

7. The osteogenic biomaterial according to claim 6, wherein the polymerizing group is an acryloyl group.

8. The osteogenic biomaterial according to any of claims 6 or 7, wherein the nanogel is cross-linked with a water-soluble polymer having a thiol group.

9. The osteogenic biomaterial according to claim 8, wherein the water-soluble polymer having a thiol group is polyethyleneoxide.

10. The osteogenic biomaterial according to any of claims 1-9, wherein the osteogenesis promoting substance has a weight ratio of approximately 6 x 10⁻⁶ - 2 x 10⁻² relative to the nanogel.

11. The osteogenic biomaterial according to any of claims 1-7, wherein the nanogel has a particle diameter of approximately 20-500 nm.

12. The osteogenic biomaterial according to any of claims 1-11, wherein the osteogenic biomaterial is a sustained release formulation.

13. The osteogenic biomaterial according to any of claims 1-11, wherein the osteogenic biomaterial is an injection, a paste, or an emulsion.

14. A bone disease preventive and/or therapeutic agent comprising an osteogenic biomaterial according to any of claims 1-11.

15. A method for producing the osteogenic biomaterial according to any of claims 1-13.
